# FASCICULE DE BREVET EUROPEEN

(11) **EP 3 019 145 B1**
(45) Date de publication et mention de la délivrance du brevet: **20.09.2017**
(21) Numéro de dépôt: 14739798.8
(22) Date de dépôt: 11.07.2014
(51) Int. Cl.: A61K 8/37, A61K 8/63, A61Q 19/00, A61K 8/97, A61K 8/06, A61Q 1/00, A61K 8/86, A61K 31/215, A61K 31/575

(54) **COMPOSITION COSMÉTIQUE COMPRENANT UNE ASSOCIATION TERNAIRE LIPIDIQUE POUR LUTTER CONTRE LA SÉCHERESSE CUTANÉE**
KOSMETISCHE ZUBEREITUNG ENTHALTEND EINER TERNÄREN LIPIDISCHEN VERBIDUNG ZUR BEHANDLUNG VON HAUTTROCKENHEIT
COSMETIC COMPOSITION COMPRISING A TERNARY LIPIDIC ASSOCIATION FOR TREATING SKIN DRYNESS

(30) Priorité: 12.07.2013 FR 1356892
(43) Date de publication de la demande: 18.05.2016
(73) Titulaire: Pierre Fabre Dermo-Cosmétique, 92100 Boulogne-Billancourt (FR); Université Paris-Sud 11, 91400 Orsay (FR); Centre Hospitalier Universitaire De Toulouse, 31300 Toulouse (FR)
(72) Inventeur: DUPLAN, Hélène, F-31320 Auzeville Tolosan (FR); REDOULES, Daniel, F-31100 Toulouse (FR); DELALLEAU, Alexandre, F-31770 Colomiers (FR)
(74) Mandataire: Regimbeau
(86) Numéro de dépôt international: PCT/EP2014/064972
(87) Numéro de publication internationale: WO 2015/004279

(56) Documents cités:
- EP-A1- 2 583 667
- FR-A1- 2 799 122
- FR-A1- 2 880 802
- US-A- 3 957 971
- US-A- 4 604 281

## Description

Le domaine de la présente invention concerne la nouvelle association ternaire lipidique suivante : bétasitostérol, isocétyl stéaroyl stéarate et glycéryl tri-2éthylhexanoate ; et ses applications dans les domaines de la cosmétique et de la dermatologie pour lutter contre la sécheresse cutanée.

Plus particulièrement, la présente invention concerne l'utilisation cosmétique d'une composition comprenant ladite association ternaire lipidique, comme agent hydratant pour la peau.

Un autre objet de la présente invention concerne en outre une composition dermatologique comprenant ladite association ternaire lipidique pour son utilisation dans le traitement de la sécheresse cutanée pathologique.

La peau constitue une interface entre l'organisme et l'environnement extérieur et à ce titre, elle est non seulement protectrice vis-à-vis de la pénétration d'éléments chimiques ou microbiens mais elle doit aussi assurer le maintien du milieu physiologique de l'organisme en limitant les déperditions hydriques. Sur ce dernier point, la couche la plus superficielle de l'épiderme, le stratum corneum (qui sera désigné par SC dans l'ensemble de la présente demande de brevet), participe de façon majeure à une homéostasie osmotique dont le rôle est de maintenir un gradient hydrique et ainsi, de réduire les effets dessicatifs dus à l'environnement. Dans les conditions physiologiques, la teneur en eau dans la partie superficielle de l'épiderme varie de 70 à 30 % en passant des dernières couches vivantes (stratum granulosum) au stratum corneum. Elle baisse encore dans les toutes dernières couches cornéocytaires pour atteindre 15 %.

Cette capacité à maintenir un équilibre hydrique provient directement de la structure du stratum corneum, lequel est constitué de cellules aplaties kératinisées, les cornéocytes, empilés dans une matrice lipidique. Cette dernière composée de céramides, cholestérol et acides gras forme un empilement successif de phases lamellaires orientées de façon parallèle à la surface de la peau. La diffraction des rayons X révèle que les chaînes lipidiques sont organisées en deux phases, l'une cristalline dans un arrangement orthorombique l'autre plus fluide et moins compacte dans un arrangement hexagonal. La phase cristalline beaucoup plus compacte est prépondérante dans le stratum corneum mais la proportion de lipides dans un ordre hexagonal augmente en allant vers la superficie.

Ainsi, l'intégrité de ces structures lipidiques qui assurent l'étanchéité relative à l'eau par leur qualité hydrophobe est indispensable pour que la couche cornée puisse retenir l'eau.

Parmi les facteurs tendant à favoriser le dessèchement du stratum corneum figurent notamment l'utilisation de tensioactifs (par ex., le lauryl sulfate de sodium (LSS)) et la faible humidité de l'environnement (froid sec, chauffage...). En effet, il a été montré que l'application de LSS réduisait la proportion de lipides dans une phase orthorhombique et augmentait celle dans une phase hexagonale (Saad P, Flach CR, Walters RM, Mendelsohn R. Infrared spectroscopic studies of sodium dodecyl sulphate permeation and interaction with stratum corneum lipids in skin. Int J Cosmet Sci. 2012 ; 34 ; 36-43). Cet endommagement des structures lamellaires conduit à une augmentation de la perte insensible en eau trans-épidermique. De même, le dessèchement produit par une exposition prolongée à une atmosphère de faible hygrométrie provoque la formation d'aires cristallines autour desquelles l'eau s'échappe facilement. Dans ces différents contextes, le volume d'eau dans les couches superficielles diminue et le stratum corneum tend à se rétracter. Ses propriétés mécaniques (tension, module d'élasticité) sont modifiées (Levi K, Weber RJ, Do JQ, Dauskardt RH. Drying stress and damage processes in human stratum corneum. Int J Cosmet Sci. 2010;32: 276-93). La peau devenue dure et rugueuse se traduit par une sensation d'inconfort avec tiraillement. A un stade plus sévère, le stratum corneum perd sa qualité de barrière ce qui déclenche notamment les réponses suivantes: augmentation de la perspiration intra-épidermique, prolifération kératinocytaire excessive, modification de l'arrangement supramoléculaire de la matrice lipidique. Cliniquement, la peau devient plus épaisse et squameuse la rendant sujette aux craquelures.

Lorsqu'un patient atteint de sécheresse cutanée est pris en charge, les objectifs premiers sont d'améliorer son confort cutané et sa qualité de vie, et de ramener la couche cornée à un état homéostatique normal.

Généralement, les formulateurs utilisent certains corps gras qui stabilisent les structures lipidiques lamellaires du stratum corneum en limitant les mouvements latéraux des lipides intercornéocytaires.

Une autre alternative pour tamponner la viscosité des couches lipidiques consiste à utiliser des stérols, qui ont la propriété de limiter, en fonction des conditions environnantes de dessèchement : soit le déplacement des chaînes grasses trop fluides, soit le rapprochement des chaînes grasses trop rigides.

Un exemple de corps gras classiquement utilisé est la vaseline dont les longues chaînes en C-26 viennent se placer en interdigitation dans les bicouches céramidiques du stratum corneum. Cette action quasi occlusive induit une diminution de l'évaporation de l'eau à travers le stratum corneum. On peut obtenir des actions similaires en utilisant des esters symétriques à chaînes longues, lesquels ont l'avantage d'être moins occlusifs que la vaseline et par conséquent plus agréables à utiliser. Parmi les plus connus, on peut citer le myristyl myristate, le cétyl palmitate ou l'isostéaryl isostéarate. Pour ce dernier, des études ont pu établir qu'il empêche la transition de phase des lipides intercornéocytaires (orthorhombique à hexagonal) qui s'opère avec l'augmentation de la température (Caussin J, Gooris GS, Bouwstra JA, FTIR studies show lipophilic moisturizers to interact with stratum corneum lipids, rendering the more densely packed. Biochim Biophys Acta. 2008; 1778: 1517-24).

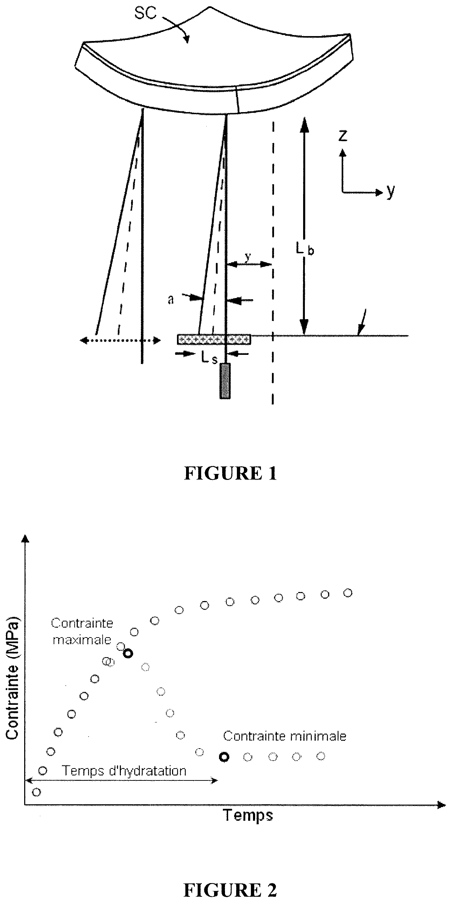

On retrouve encore dans de nombreuses formules des esters asymétriques constituées d'une longue chaine et d'une ou plusieurs chaînes ramifiées plus courtes. Ces esters cumulent les avantages : amélioration du toucher apportée par les chaînes ramifiées et rôle tampon vis-à-vis des espaces lipidiques intercornéocytaires. Un travail effectué sur du stratum corneum isolé montre l'effet protecteur qu'exercent certains esters asymétriques vis-à-vis des contraintes mécaniques associées à un stress de déshydratation (Levi et al, Int J Cosmet Sci. 2010;32: 276-93). En effet, dans cette étude, les résultats indiquent notamment une bonne efficacité de l'isocétyl stéaroyl stéarate vis-à-vis de la rétractation de l'échantillon de stratum corneum desséché par un manque d'humidité. Cependant, il a été également observé que les dérivés possédant des chaînes grasses volumineuses tel que l'isocétyl stéaroyl stéarate pouvait conduire à une désorganisation plus ou moins temporaire des structures lipidiques intercornéocytaires avant d'exercer leur effet stabilisateur des structures lamellaires intercornéocytaires. On le voit notamment sur la figure 1, où l'application topique isocétyl stéaroyl stéarate majore, durant les 4 premières heures, la rétractation cutanée induite par le stress desséchant.

Aussi il apparaît nécessaire de disposer d'une composition hydratante qui ne présente pas les inconvénients associés à l'utilisation de ces esters asymétriques à chaîne longue.

Le but de la présente invention est d'apporter une contribution originale aux traitements des peaux sèches.

Au cours de leur recherche, les inventeurs ont mis en évidence qu'en ajoutant à l'ester isocétyl stéaroyl stéarate une quantité de β sitostérol égale ou inférieure à 5% en poids par rapport à l'ester, on améliore nettement la protection contre la déshydratation.

Il a en effet été montré que dans l'approche biomécanique du SC consistant en l'évaluation de l'état de contrainte du SC isolé déshydraté, l'association de l'isocétyl stéaroyl stéarate avec le β sitostérol permet d'améliorer les 3 critères retenus (Cf figure 2), à savoir diminuer la contrainte maximale, la contrainte minimale et le temps d'hydratation.

De façon surprenante et inattendue, il a été observé au cours de l'étude biomécanique du SC, que l'ajout à l'ester isocétyl stéaroyl stéarate et au β sitostérol, d'un troisième corps gras cosmétique, le tri-2-éthylhexanoate, s'accompagne d'une diminution d'une part du temps nécessaire pour atteindre l'équilibre et d'autre part, de la contrainte qui s'établit à l'issu de ce temps.

Ainsi, la ramification sur ces triglycérides à chaîne courte est indispensable pour que l'association des trois corps gras, isocétyl stéaroyl stéarate, de β sitostérol et de glycéryl tri-2-éthylhexanoate, puisse permettre un fonctionnement optimisé des espaces lipidiques intercornéocytaires dans un environnement desséchant.

La présente invention concerne donc l'association ternaire lipidique suivante : isocétyl stéaroyl stéarate, β sitostérol et de glycéryl tri-2-éthylhexanoate, et plus spécifiquement l'association ternaire dans laquelle le bétasitostérol représente de 0,1 % à 5 % en poids de l'association.

Selon une caractéristique additionnelle de la présente invention, l'isocétyl stéaroyl stéarate et le glycéryl tri-2éthylhexanoate sont présents dans ladite association ternaire dans des proportions massiques sensiblement égales.

Avantageusement, l'association ternaire lipidique selon l'invention consiste en :
- 5% de β-sitostérol ;
- 47,5 % d'isocétyl stéaroyl stéarate, et
- 47,5 % de glycéryl tri-2éthylhexanoate,
les pourcentages en poids étant exprimés par rapport au poids total de ladite association.

Un autre objet de la présente invention vise une composition émolliente comprenant de 1 à 20 % de ladite association ternaire ; les pourcentages étant exprimés en poids par rapport au poids total de ladite composition.

### PRESENTATION DES FIGURES :

Les figures 1 à 3 concernent le modèle d'étude de l'état de contrainte du SC isolé déshydraté
   - la figure 1 présente le principe de mesure de l'évolution des contraintes après application d'un émollient,
   - la figure 2 présente les 3 critères d'analyse qui seront discutés : contrainte maximale, contrainte minimale et temps d'hydratation (temps d'action pour atteindre l'équilibre)
   - la figure 3 représente les résultats obtenus pour la série suivante :
      - témoin,
      - Isocétyl Stéaroyl Stéarate (ISS) = 1,
      - Bétasitostérol + ISS = B1,
      - Bétasitostérol + ISS + glycérol 2 ethylhexanoate = B1T.

La déshydratation du SC est associée à divers mécanismes tels que : l'augmentation des forces intermoléculaires entre lipides intercellulaires, la modification des chaînes des kératines et une réduction du volume en eau du SC. Ces phénomènes conduisent à la modification de ses propriétés biophysiques : épaisseur, élasticité et tension - qui elles-mêmes ont une conséquence vitale sur son intégrité.

S'appuyant sur les données cliniques macroscopiques associées à la sécheresse cutanée, et en considérant le SC comme un film de surface et un matériau, il a été proposé que sensations et aspects du SC soit directement reliés à ses propriétés mécaniques. Ainsi il est possible de décrire et mesurer les sensations d'inconfort et de tension au niveau cutané, ou un aspect craquelé du SC, par des descripteurs biomécaniques du SC.

Ces éléments ont conduit la Demanderesse à utiliser un modèle in vitro de SC isolé permettant d'étudier les modifications de ses propriétés mécaniques en fonction de son niveau d'hydratation. La mesure de tension cutanée permet de définir une relation entre l'état de sécheresse cutanée et le niveau de contrainte cutanée ainsi que l'apparition de craquelures. En outre, l'utilisation de ce modèle permet de connaître avec une grande précision l'impact d'actifs sur ces mesures mécaniques, et par conséquent, sur l'état d'hydratation du stratum corneum. (K. Levi, R. J. Weber, J. Q. Do and R. H. Dauskardt.International Journal of Cosmetic Science, 2010, 32, 276-293 Drying stress and damage processes in human stratum corneum) (K. Levi and R. H. Dauskardt.International Journal of Cosmetic Science, 2010, 32, 294-298 Application of substrate curvature method to differentiate drying stresses in topical coatings and human stratum corneum).

La composition selon l'invention permet une diminution de la contrainte ou tension cutanée, et ainsi une amélioration des sensations de tiraillements de la peau et diminution des rugosités, craquelures et/ou crevasses.

Dans un mode de réalisation particulier de l'invention, la composition comprend en outre un ou plusieurs autres humectants pour compléter l'action de l'association ternaire lipidique.

De façon générale, toutes les approches visant à renforcer l'aspect fonctionnel des lipides intercornéocytaires dans des conditions desséchantes sont généralement combinées dans des émulsions avec des humectants de nature hygroscopique tels que le glycérol, l'urée, du sorbitol et d'autres encore.

Dans un autre mode de réalisation particulier de l'invention, la composition comprend en outre un ou plusieurs autres principes actifs cosmétiques ou dermatologiques.

On peut citer à titre d'exemple, le di(guanosine-5')tétraphosphate (GP4G), principe actif de biotechnologie marine extrait de zooplancton artemia Salina, connu pour son action stimulante de l'activité mitochondriale et du métabolisme cellulaire.

Préférentiellement, les compositions selon l'invention seront administrées par voie topique.

Plus préférentiellement, les compositions selon l'invention se présentent sous forme d'émulsion.

Il pourra s'agir de produits de soin et/ou de maquillage pour la peau du corps et/ou du visage.

Un autre aspect de la présente invention concerne l'utilisation cosmétique de la composition selon l'invention comme agent hydratant pour la peau.

Plus particulièrement, ladite composition est destinée à hydrater l'épiderme, en particulier la couche cornée. La présente invention vise également un procédé cosmétique d'hydratation de la peau comprenant l'application sur la peau d'une composition émolliente selon l'invention.

C'est enfin un autre objet de la présente invention que de fournir une composition dermatologique comprenant ladite association ternaire lipidique pour son utilisation dans le traitement de la sécheresse cutanée pathologique.

Par « sécheresse cutanée pathologique », on entend au sens de la présente invention, tout type de sécheresse cutanée, soit directement liée à une pathologie de la peau, soit résultant du traitement dermatologique de cette dernière.

Plus particulièrement, ladite composition est destinée à améliorer les symptômes de toute forme de sécheresse cutanée pathologique, en termes d'aspect tel que la rugosité ou de perceptions associées (prurit, tiraillement).

### EXEMPLES

**Soin hydratant:**

| **INCI désignation** | **Pourcentage** | **Fonction** |
|---|---|---|
| Eau purifiée | QSP 100% | |
| Glycérine | 3 | Humectant |
| Disodium EDTA | 0.1 | Agent complexant |
| Phénoxyéthanol | 0.35 | Conservateur |
| Polyacrylate-13 & Polyisobutène & Polysorbate 20 & Water | 1 | Gélifiant, agent stabilisant |
| Glycéryl Stéarate & PEG-100 Stérate | 4 | Emulsionnant |
| Cétyl Alcohol | 1 | Facteur de consistance |
| Caprylic/Capric Triglycérides | 6 | Emollient |
| Paraffinium Liquidium | 4 | Emollient |
| Dicaprylyl Carbonate | 4 | Emollient |
| Association ternaire lipidique (B 5% - 1 47,5% - T 47,5%) | **3%** | Actif |
| Parfum | 0.1 | Parfum |

**Sérum d'un soin hydratant :**

| **INCI désignation** | **Pourcentage** | **Fonction** |
|---|---|---|
| Eau | QSP 100% | |
| Glycérine | 3 | Humectant |
| Disodium EDTA | 0.1 | Agent complexant |
| Glycol | 0.3 | Humectant |
| | | |
| Sodium Polyacrylate | 1 | Gélifiant, agent stabilisant |
| | | |
| Diméthicone | 4 | Emollient |
| Myristyl Alcohol & Myristyl glucoside | 2 | Facteur de consistance |
| Acide benzoique | 0.3 | Conservateur |
| Association ternaire lipidique (B 5% - 1 47,5% - T 47,5%) | **6%** | Actif |
| | | |
| Parfum | 0.1 | Parfum |

### EVALUATION :

L'approche mécanique utilisée ici, consiste à mesurer les contraintes mécaniques induites au sein d'un SC isolé et soumis à un stress de déshydratation dans une chambre environnementale.

L'échantillon de SC issu d'explants de peau humaine a été isolé par digestion enzymatique. Il a été placé sur une lame de borosilicate à la quelle il adhère naturellement

Le SC hydraté initialement à 100% est ensuite placé dans une atmosphère à un taux d'hygrométrie de 7%. Le SC subit ainsi une déshydratation progressive, pendant laquelle des mesures de tension du SC sont réalisées toutes les 15 min pendant 8 heures à l'aide d'un appareil de mesure de contraintes. Lors de la déshydratation, en raison notamment de sa perte de volume d'eau, le stratum corneum se rétracte. Compte tenu des différences existant entre ses propriétés mécaniques et celles du support (lame), ce dernier se courbe. Par l'intermédiaire de ce rayon de courbure, mesuré grâce à un interféromètre laser, il est alors possible de calculer l'état de tension/contrainte à partir de l'équation de Stoney. Les données initiales correspondent à une mesure de variation de courbure de l'ensemble (lame de borosilicate + SC). Le calcul des paramètres biomécaniques de contrainte du SC, tels que la tension, est possible de par la connaissance des propriétés biomécaniques de la lame de borosilicate de son épaisseur ainsi que de l'épaisseur du SC (Cf méthodologie Figure 1 ; et référence bibliographique K. Levi, et al, IJCS 2010*).*

Cette première cinétique permet de connaître le comportement du SC non traité. Après celle-ci, l'ensemble (SC+lame) est remis à un taux d'hygrométrie de 100% pendant 2h puis mis à incuber dans les différents traitements pendant 5 min. Un film de 50µm est déposé : à l'aide d'un filmographe à spirale pour les émollients fluides et à l'aide d'une lame de dépôt en ce qui concerne les crèmes. A l'issue de ce temps, l'ensemble est de nouveau placé à un taux d'hygrométrie de 7% pour une cinétique de déshydratation jusqu'à obtention d'une stabilisation des mesures (de 8 à 12h généralement). La cinétique de développement des contraintes au sein du SC soumis au stress de déshydratation est réalisée et comparée au contrôle « Eau » qui correspond au non traité.

### Descriptions de la Composition des mélangea :

1 : 100% d'Isocétyl Stéaroyl Stéarate (ISS)
B1 : Bétasitostérol (5%) + ISS (95%)
B1T : Bétasitostérol (5%) + ISS (47.5%) + glyceryl tri-2ethylhexanoate (47.5%)

### Interprétation des courbes, conclusion :

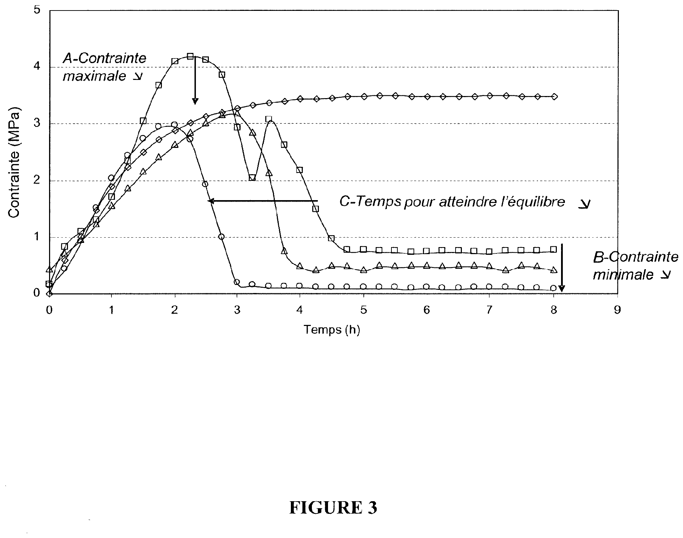

Dans la figure 3, le témoin correspond au SC non traité avant la cinétique de déshydratation (-0-). Sont représentées les mesures de contraintes mécaniques au sein du SC (MPa) en fonction de la cinétique de déshydratation (h). Ces contraintes augmentent avec la chute du taux d'hygrométrie, en fonction du temps, jusqu'à atteindre un plateau.

Lorsque le SC est prétraité par l'ISS 100% (1 - -□-), la courbe met en évidence une contrainte maximale momentanément supérieure au contrôle mais qui chute rapidement (à partir de 3h de déshydratation) pour atteindre un plateau de contrainte minimale après 2h.

Lorsque l'ISS est complété par 5% de betasitostérol (B1 - -Δ-) puis par 47.5% de glycéryl tri-2éthylhexanoate, le plateau de contrainte minimal est encore diminué et est atteint plus rapidement.

L'association sous forme de trio est la plus efficace pour limiter la génération de contraintes mécaniques au sein du SC en cours de dessèchement (B1T - -○-).

Il a ainsi été montré que cette association empêche la rétractation de la couche cornée en condition de sécheresse tout en modérant la déstabilisation des espaces lipidiques que génère l'ester isocétyl stéaroyl stéarate. En comparant les courbes de la figure 3, on constate bien que l'ajout de 5% en poids de β sitostérol à l'ester asymétrique non seulement évite l'excès de contrainte dû à l'encombrement de l'ester mais encore, permet d'abaisser cette dernière à un niveau significativement plus bas à l'état d'équilibre. Dans notre modèle expérimental, cet état d'équilibre est atteint au bout d'un intervalle de temps compris entre 3 et 5 heures selon les traitements et correspond vraisemblablement au temps nécessaire à la pénétration des substances émollientes dans les espaces lipidiques intercornéocytaires pour exercer une stabilisation de ces derniers.

Pour des raisons de solubilité, la proportion de β-sitostérol au sein de ladite association ne doit pas être supérieure à 5%. Cette valeur de 5% apparaît par conséquent comme le meilleur compromis concernant l'efficacité d'un produit.

L'utilisation de la composition B1T [ISS (47.5%), β-sitostérol (5%), glycéryl tri-2éthylhexanoate (47.5%)] permet d'atteindre le minimum des contraintes plus rapidement qu'une composition B1 ou 1 (Fig 3).

## Revendications

1. Association ternaire lipidique consistant en β-sitostérol, isocétyl stéaroyl stéarate et glycéryl tri-2éthylhexanoate, dans laquelle le β-sitostérol est présent à raison de 0,1 % à 5 % en poids de ladite association.

2. Association ternaire lipidique selon la revendication 1, **caractérisée en ce que** l'isocétyl stéaroyl stéarate et le glycéryl tri-2éthylhexanoate sont présents dans des proportions massiques sensiblement égales.

3. Association ternaire lipidique selon l'une des revendications 1 ou 2, **caractérisée en ce qu'**elle présente la composition suivante :
- 5 % de β-sitostérol
- 47,5 % d'isocétyl stéaroyl stéarate, et
- 47,5 % de glycéryl tri-2éthylhexanoate
les pourcentages étant exprimés en poids par rapport au poids total de ladite association.

4. Composition émolliente comprenant de 1 à 20 % de l'association telle que définie dans l'une des revendications 1 à 3 ; les pourcentages étant exprimés en poids par rapport au poids total de ladite composition.

5. Composition selon la revendication 4 **caractérisée en ce qu'**elle comprend en outre un ou plusieurs autres agents humectants.

6. Composition selon l'une des revendications 4 ou 5 **caractérisée en ce qu'**elle comprend en outre un autre actif cosmétique, et en particulier le di(guanosine-5')tetraphosphate (GP4G).

7. Composition selon l'une quelconque des revendications 4 à 6 **caractérisée en ce qu'**elle se présente sous la forme d'une émulsion.

8. Composition selon l'une quelconque des revendications 4 à 7, **caractérisée en ce qu'**elle se présente sous la forme d'un produit de soin et/ou de maquillage de la peau du corps ou du visage.

9. Composition selon l'une quelconque des revendications 4 à 8 pour son utilisation comme agent hydratant pour la peau.

10. Composition selon la revendication 9 pour son utilisation comme agent hydratant de l'épiderme, en particulier la couche cornée.

11. Composition selon l'une quelconque des revendications 4 à 8 pour son utilisation dans le traitement de la sécheresse cutanée pathologique.

12. Composition selon la revendication 11 **caractérisée en ce que** la composition est destinée à améliorer les symptômes de toute forme de sécheresse cutanée pathologique.

## Patentansprüche

1. Ternäre Lipidverbindung, bestehend aus β-Sitosterol, Isocetylstearoylstearat und Glyceryl-tri-2ethylhexanoat, wobei β-Sitosterol im Umfang von 0,1 Gew% bis 5 Gew% der Verbindung vorhanden ist.

2. Ternäre Lipidverbindung nach Anspruch 1, **dadurch gekennzeichnet, dass** Isocetylstearoylstearat und Glyceryl-tri-2ethylhexanoat in im Wesentlichen gleichen Massenproportionen vorhanden sind.

3. Ternäre Lipidverbindung nach Anspruch 1 oder 2, **dadurch gekennzeichnet, dass** sie die folgende Zusammensetzung aufweist:
- 5 % β-Sitosterol
- 47,5 % Isocetylstearoylstearat und
- 47,5 % Glyceryl-tri-2ethylhexanoat,
wobei die Prozentsätze in Gewicht mit Bezug auf das Gesamtgewicht der Verbindung ausgedrückt sind.

4. Weichmacherzusammensetzung, umfassend von 1 bis 20 % der Verbindung, wie in einem der Ansprüche 1 bis 3 definiert; wobei die Prozentsätze in Gewicht mit Bezug auf das Gesamtgewicht der Zusammensetzung ausgedrückt sind.

5. Zusammensetzung nach Anspruch 4, **dadurch gekennzeichnet, dass** sie außerdem ein oder mehrere weitere Befeuchtungsmittel umfasst.

6. Zusammensetzung nach einem der Ansprüche 4 oder 5, **dadurch gekennzeichnet, dass** sie außerdem einen weiteren kosmetischen Wirkstoff umfasst und insbesondere Di(guanosin-5')tetraphosphat (GP4G).

7. Zusammensetzung nach einem beliebigen der Ansprüche 4 bis 6, **dadurch gekennzeichnet, dass** sie die Form einer Emulsion aufweist.

8. Zusammensetzung nach einem beliebigen der Ansprüche 4 bis 7, **dadurch gekennzeichnet, dass** sie die Form eines Pflege- und/oder Make-up-Produkts der Haut des Körpers oder des Gesichts aufweist.

9. Zusammensetzung nach einem beliebigen der Ansprüche 4 bis 8 für ihre Verwendung als Feuchthaltemittel für die Haut.

10. Zusammensetzung nach Anspruch 9 für ihre Verwendung als Feuchthaltemittel der Epidermis, insbesondere der Hornschicht.

11. Zusammensetzung nach einem beliebigen der Ansprüche 4 bis 8 für ihre Verwendung bei der Behandlung der pathologischen Trockenheit der Haut.

12. Zusammensetzung nach Anspruch 11, **dadurch gekennzeichnet, dass** die Zusammensetzung ausgelegt ist, um die Symptome jeder Form von pathologischer Trockenheit der Haut zu verbessern.

## Claims

1. Ternary lipid association consisting of β-sitosterol, isocetyl stearoyl stearate and glyceryl tri-2-ethylhexanoate, wherein β-sitosterol is present in a proportion of 0.1% to 5% by weight of said association.

2. Ternary lipid association according to claim 1, **characterized in that** isocetyl stearoyl stearate and glyceryl tri-2-ethylhexanoate are present in substantially equal mass proportions.

3. Ternary lipid association according to one of claims 1 or 2, **characterized in that** it has the following composition:
- 5% of β-sitosterol
- 47.5% of isocetyl stearoyl stearate, and
- 47.5% of glyceryl tri-2-ethylhexanoate
the percentages being expressed by weight relative to the total weight of said association.

4. Emollient composition comprising from 1% to 20% of the association as defined in one of claims 1 to 3, the percentages being expressed by weight relative to the total weight of said composition.

5. Composition according to claim 4 **characterized in that** it further comprises one or more other humectants.

6. Composition according to one of claims 4 or 5 **characterized in that** it further comprises another cosmetic active agent, and in particular di(guanosine-5')tetraphosphate GP4G.

7. Composition according to any one of claims 4 to 6 **characterized in that** it takes the form of an emulsion.

8. Composition according to any one of claims 4 to 7, **characterized in that** it takes the form of a skincare and/or makeup product for the body or face.

9. Composition according to any one of claims 4 to 8 for use as moisturizer for the skin.

10. Composition according to claim 9 for use as moisturizer for the epidermis, in particular the stratum corneum.

11. Composition according to any one of claims 4 to 8 for use in the treatment of pathological dry skin.

12. Composition according to claim 11 **characterized in that** the composition is for improving the symptoms of any form of pathological dry skin.
